# EUROPÄISCHE PATENTANMELDUNG

(11) **EP 2 907 828 A1**
(43) Veröffentlichungstag der Anmeldung: **19.08.2015**
(21) Anmeldenummer: 14155125.9
(22) Anmeldetag: 14.02.2014
(51) Int. Cl.: C08F 2/08, A61K 8/00, C08F 226/06, C08F 226/10

(54) **Verfahren zur Herstellung von kationogen-ampholytischen VP/VI-Copolymeren**

(71) Anmelder: BASF SE, 67056 Ludwigshafen (DE)
(72) Erfinder: Nguyen-Kim, Son, 69502 Hemsbach (DE); Schade, Christian, 67063 Ludwigshafen (DE); Hössel, Peter, 67105 Schifferstadt (DE); Werner, Rolf, 67063 Ludwigshafen (DE)
(74) Vertreter: Reinhardt, Jürgen

(57) **Zusammenfassung**

Die vorliegende Erfindung betrifft ein Verfahren zur Herstellung eines kationischampholytischen Vinylpyrrolidon-Copolymer, dadurch gekennzeichnet, dass das Vinylpyrrolidon-Copolymer in einem wasserfreien, kosmetischen Öl oder in einem kosmetischen Öl/Essigesser-Gemisch durch Fällungspolymerisation in Form einer Öl-Dispersion erhalten wird.

## Beschreibung

Die vorliegende Erfindung betrifft ein Verfahren zur Herstellung eines kationisch-ampholytischen Vinylpyrrolidon-Copolymer, dadurch gekennzeichnet, dass das Vinylpyrrolidon-Copolymer in einem wasserfreien, kosmetischen Öl oder in einem kosmetischen Öl/Essigesser-Gemisch durch Fällungspolymerisation in Form einer Öl-Dispersion erhalten wird.

Pulverförmige rheologische Modifizierungsmitteln sind bekannt und haben in vielen Bereichen (Anstrich-, Papier-, Textil-, Hygiene-, Kosmetik- und nicht zuletzt in Pharma-Industrien) ihre Anwendung gefunden.

Einer der derzeitig wichtigsten Verdicker ist Carbopol^{®}, eine vernetzte anionische Polyacrylsäure der Firma Lubrizol (früher Goodrich). Unterschiedliche Carbopol-Typen sowie Herstellungsverfahren werden beansprucht in US 2798053**,** US 3915921**,** US 3940351**,** US 6433061**,** US 4062817**,** EP 328725**,** EP 584771**,** EP 279892**.**

Auch Verdicker auf Basis von anionisch-ampholytischen VP-Copolymeren sind bekannt, siehe WO 2004/58837**.** Alle Acrylsäure-basierende Verdicker werden bevorzugt durch Fällungspolymerisation hergestellt und in Form eines Pulvers erhalten.

Kationische/kationogene VP-Coplymere sind bekannt, sie werden in Wasser durch Lösungspolymerisation hergestellt oder in Ethanol/Cyclohexan durch Fällungspolymerisation hergestellt, siehe US 5608021**.** Kationisch assoziative Verdicker können auch durch Emulsionspolymerisation hergestellt werden, siehe US 2004/52746**.**

Kation-ampholytische VP-Copolymere sind auch bekannt, siehe WO 00/39176 (Goodrich), WO 04/58837 **(BASF),** WO 07/10034 **(BASF)** WO 07/10035 **(BASF).** Typisches Beispiel für die kation-ampholytische Stoffklasse ist Luvigel^{®} Advanced, was als filmbildender Verdicker mit konditionierender Eigenschaft zur Formulierung von Gelen sowie Emulsionen eingesetzt wird. Aufgrund der Hydrophobie und kationischen Natur sind solche Polymere besonders gut geeignet für die Formulierung von kosmetischen Präparaten bei pH = 4 - 7, insbesondere in Shampoos, Duschbäder und Emulsionen.

Die oben genannten kationischen Copolymere werden bevorzugt mittels einer kostspieligen, konventionellen Fällungspolymerisation hergestellt, wobei als Lösungsmittel Cyclohexan und/oder Essigester eingesetzt wird. Das von dem Lösungsmittel ausgefallende Produkt wird filtriert und dann in einem ex-geschützten Trockenschrank unter Vakuum getrocknet.

WO 99/29735 offenbart die Herstellung von Polyvinylpyrrolidon und von Copolymeren aus Vinylpyrrolidion und anderen Monomeren. Dabei werden die Monomere in einem Öl, zum Beispiel einem Silikonöl oder einem Mineralöl, gelöst und das sich bildende Polymer fällt aus diesem Öl aus, so dass eine Dispersion des Polymeren in Öl erhalten wird. Als Comonomere werden gemäß der Beispiele Laurylmethacrylat und Acrylsäure verwendet.

Der vorliegenden Erfindung lag die Aufgabe zugrunde, ein kostengünstiges Verfahren zur Herstellung von kation-ampholytischen VP/VI-MAS-Coplymeren zu entwickeln.

Die Lösung dieser Aufgabe und damit die vorliegende Erfindung ist ein Verfahren zur Herstellung von kationisch ampholytischen VP-Copolymeren als Rheology-Modifier und als Polymer Emulgatoren zur Formulierung von tensid-haltigen Präparaten und Emulsionen, dadurch gekennzeichnet, dass das VP-Copolymer in einem wasserfreien kosmetischen Öl oder kosmetischem Öl/ Essigesser-Gemisch durch Fällungspolymerisation in Form einer Öl-Dispersion (evtl. nach Abdestillieren von Essigester) erhalten werden kann. Dadurch können die beiden Aufarbeitungsschritte, nämlich das Filtrieren und das Trocknen erspart werden. Ein weiterer Vorteil ist, dass sich das Polymer (in Form einer Dispersion) sehr einfach formulieren lässt.

### Beispiele

Folgende VP-Copolymere werden in einem kosmetischen Öl, wie z.B. Waglinol^{®} 2/7680, Myristol^{®} 318 ... polymerisiert und in Form einer Dispersion hergestellt:

| **Labor-Journal Nummer** | **VP** | **VI** | MAS | LUMA | Acrylat: MMA o SMA | PETAE [%] ppmH | Emulgator | FG[%] in Waglinol 2/7680 | FG[%] ir Myristol 318 |
|---|---|---|---|---|---|---|---|---|---|
| **GK 2832-233** | **56.8** | **36** | 3.6 | 3.6 | -- | 0.15 | Emulgade 1% + 1%Belsil | 20% | -- |
| **GK 2832-234** | **56.8** | **36** | 3.6 | 3.6 | -- | 0.15 | Belsil DMC 6031 1% | 20% in Waglinol+ EE // EE wird abdestill. | -- |
| **GK 2832-246** | **56,8** | **36** | 3.6 | 3.6 | -- | 0.15 | Emulgade 10% | **20%** in Waglinol | -- |
| **GK 2832-237 GK 2832-238** | **38** | **50** | 6 | 6 | -- | 0.3 | Emulgade 5% | 20% in Waglinol+ EE // EE wird abdestill. | -- |
| **GK 2832-240** | **38** | **50** | 6 | 6 | -- | 0.3 | Emulgade 5% | **28%** in Waglinol | -- |
| **GK 2832-241** | | | | | | | | | |
| **GK 2832-243** | **38** | **50** | 6 | 6 | -- | 0.3 | Emulgade 5% | **20%** in Waglinol | -- |
| **GK 2832-244** | **38** | **50** | 6 | 6 | -- | 0.3 | Emulgade 5% | **20%** in Waglinol : Luvito 50:50 | -- |
| **GK 2832-244** | **38** | **50** | 6 | 6 | -- | 0.15 | Emulgade 10% | **20%** in Waglinol | -- |
| **GK 2832-247** | **38** | **50** | 6 | 6 | -- | 0.30 | Emulgade 10% | **20%** in Waglinol | -- |
| **GK 10046-239** | **56,8** | **36** | 3.6 | 3.6 | -- | 0.15 | Span 85 10% | -- | 25% |
| **GK 10046-244** | **56,8** | **36** | 3.6 | 3.6 | -- | 0.30 | Span 85 10% | -- | 25% |
| **GK 10046-247** | **26,8** | **36** | 3.6 | 3.6 | MMA 30 | 0.30 | Span 85 10% | -- | 22% |
| **GK 10046-251** | **26,8** | **36** | 3.6 | 3.6 | MMA 30 | 0.15 | Span 85 10% | -- | 20% |
| **GK 10046-311** | **46,8** | **36** | 3.6 | 3.6 | SMA 10 | 0.30 | Span 85 1% | -- | 25% |
| **GK 10046-312** | **41,8** | **36** | 3.6 | 3.6 | SMA 15 | 0.30 | Span 85 1% | -- | 25% |
| **GK 10046-325** | **36,8** | **36** | 3.6 | 3.6 | SMA 20 | 0.30 | Span 85 1%% | -- | 25% |

| | | | | | | | | | |
|---|---|---|---|---|---|---|---|---|---|
| *VI Vinylimidazol (M)AS (Meth)Acrylsäure* *VP Vinylpyrrolidon LUMA C₁₈-Alkyl-(EO)₂₅-Methacrylat* *MMA Methylmethacrylat SMA Stearylmethacrylat* *PETAE Pentaerrythrit-triallylether. Belsil^{®} Belsil^{®} DMC 6031* *Emulgade Emulgade PL 68*/*50 Span^{®} 85 Sorbitan Trioleate* *Waglinol^{®} Waglinol 2*/*7680* *EE Essigester* *Myristol^{®} Myristol 318 Luvitol^{®} Lite Hydrogenated Polyisobutene* | | | | | | | | | |

Solche Copolymere funktionieren als Rheologie-Modifizierungsmittel, Stabilisator und als Polymer Emulgatoren für Öl- und Tensid-haltige Formulierungen, insb. für Shampoos, Duschbäder und Emulsionen.

## Patentansprüche

1. Ein Verfahren zur Herstellung eines kationisch-ampholytischen Vinylpyrrolidon-Copolymer, **dadurch gekennzeichnet, dass** das Vinylpyrrolidon-Copolymer in einem wasserfreien, kosmetischen Öl oder in einem kosmetischen Öl/Essigesser-Gemisch durch Fällungspolymerisation in Form einer Öl-Dispersion (optional nach Abdestillieren des Essigesters) erhalten wird.

2. Das Verfahren nach Anspruch 1, wobei das Verfahren in einem wasserfreien, kosmetischen Öl durchgeführt wird.

3. Das Verfahren nach Anspruch 2, wobei das wasserfreie, kosmetische Öl das Triglycerid eines Octansäure-Decansäure-Gemisches oder der Ester des Propylenglykol mit einem Octansäure-Decansäure-Gemisch ist.

4. Das Verfahren nach einem der Ansprüche 1 bis 3, wobei das Vinylpyrrolidon-Copolymer aus Vinylpyrrolidon und weiteren Monomeren hergestellt wird, wobei die weiteren Monomere ausgewählt sind aus der Gruppe bestehend aus Vinylimidazol, Methacrylsäure, einem C18-Alkyl-(EO)25-Methacrylat, Methylmethacrylat, Stearylmethacrylat und Pentaerythrit-triallylether.

5. Das Verfahren nach einem der Ansprüche 1 bis 4, wobei das Vinylpyrrolidon-Copolymer aus Vinylpyrrolidon, Vinylimidazol, Methacrylsäure, einem C18-Alkyl-(EO)25-Methacrylat und Pentaerythrit-triallylether hergestellt wird.

6. Das Verfahren nach Anspruch 5, wobei das Vinylpyrrolidon-Copolymer hergestellt wird aus
38 bis 56,8 Gewichtsteile Vinylpyrrolidon,
36 bis 50 Gewichtsteile Vinylimidazol,
3,6 bis 6 Gewichtsteile Methacrylsäure,
3,6 bis 6 Gewichtsteile C18-Alkyl-(EO)25-Methacrylat und
0,15 bis 0,3 Gewichtsteile Pentaerythrit-triallylether.

7. Das Verfahren nach einem der Ansprüche 1 bis 4, wobei das Vinylpyrrolidon-Copolymer aus Vinylpyrrolidon, Vinylimidazol, Methacrylsäure, einem C18-Alkyl-(EO)25-Methacrylat, Methylmethacrylat und Pentaerythrit-triallylether hergestellt wird.

8. Das Verfahren nach Anspruch 7, wobei das Vinylpyrrolidon-Copolymer hergestellt wird aus
26,8 Gewichtsteile Vinylpyrrolidon,
36 Gewichtsteile Vinylimidazol,
3,6 Gewichtsteile Methacrylsäure,
3,6 Gewichtsteile C18-Alkyl-(EO)25-Methacrylat,
30 Gewichtsteile Methylmethacrylat und
0,15 bis 0,3 Gewichtsteile Pentaerythrit-triallylether.

9. Das Verfahren nach einem der Ansprüche 1 bis 4, wobei das Vinylpyrrolidon-Copolymer aus Vinylpyrrolidon, Vinylimidazol, Methacrylsäure, einem C18-Alkyl-(EO)25-Methacrylat, Stearylmethycrylat und Pentaerythrit-triallylether hergestellt wird.

10. Das Verfahren nach Anspruch 9, wobei das Vinylpyrrolidon-Copolymer hergestellt wird aus
36,8 bis 46,8 Gewichtsteile Vinylpyrrolidon,
36 Gewichtsteile Vinylimidazol,
3,6 Gewichtsteile Methacrylsäure,
3,6 Gewichtsteile C18-Alkyl-(EO)25-Methacrylat,
10 bis 20 Gewichtsteile Stearylmethacrylat und
0,3 Gewichtsteile Pentaerythrit-triallylether.

11. Das Verfahren nach einem der Ansprüche 1 bis 10, wobei die Herstellung des Vinylpyrrolidon-Copolymer in Gegenwart eines Emulgators durchgeführt wird.

12. Das Verfahren nach Anspruch 10, wobei der Emulgators in einer Menge von 1 bis 10 Gew.-%, bezogen auf alle eingesetzten Stoffe, eingesetzt wird.

13. Das Verfahren nach einem der Ansprüche 1 bis 12, wobei die Herstellung des Vinylpyrrolidon-Copolymer so durchgeführt wir, dass eine Dispersion mit einem Feststoffgehalt von 20 bis 25 Gew.-% entsteht.
